# DEMANDE DE BREVET EUROPEEN

(11) **EP 1 532 970 A2**
(43) Date de publication de la demande: **25.05.2005**
(21) Numéro de dépôt: 04292745.9
(22) Date de dépôt: 19.11.2004
(51) Int. Cl.: A61K 7/13

(54) **Utilisation de dérivés de triarylméthane pour la coloration de fibres kératiniques**

(30) Priorité: 21.11.2003 FR 0350883
(71) Demandeur: L'OREAL, 75008 Paris (FR)
(72) Inventeur: Plos, Grégory Gluck Heim Yoga, Tokyo 158-0097 (JP); Gawtrey, Jonathan, 92100 Boulogne (FR); Kravtchenko, Sylvain, 92600 Asnieres (FR)
(74) Mandataire: Fevrier, Murielle

(57) **Abrégé**

La présente invention a pour objet l'utilisation pour la coloration des fibres kératiniques, et en particulier des fibres kératiniques humaines telles que les cheveux, d'une composition comprenant au moins un composé comportant un cycle pouvant subir une ouverture en formant un groupement acide et / ou le colorant correspondant à ce composé dont le cycle est ouvert.

La présente invention permet en particulier d'obtenir une coloration des fibres kératiniques avec des reflets intenses et tenaces pouvant être modifiée, effacée et reformée plusieurs fois sans perte de couleur.

## Description

La présente invention a pour objet l'utilisation pour la coloration des fibres kératiniques, et en particulier des fibres kératiniques humaines telles que les cheveux, d'une composition comprenant au moins un composé comportant un cycle pouvant subir une ouverture en formant un groupement acide et / ou le colorant correspondant à ce composé dont le cycle est ouvert.

Il est connu de teindre les fibres kératiniques, et en particulier les fibres kératiniques humaines telles que les cheveux, avec des compositions tinctoriales contenant des précurseurs de colorant d'oxydation, appelés généralement bases d'oxydation, tels que des ortho- ou paraphénylènediamines, des ortho- ou para-aminophénols et des composés hétérocycliques tels que des dérivés de diaminopyrazole. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diamines aromatiques, les méta-aminophénols, les méta-diphénols et certains composés hétérocycliques tels que des composés indoliques.

La variété des molécules mises en jeu au niveau des bases d'oxydation et des coupleurs permet l'obtention d'une riche palette de couleurs.

La coloration dite "permanente" obtenue grâce à ces colorants d'oxydation doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (i.e. abîmée) entre sa pointe et sa racine.

Il est aussi connu de teindre les fibres kératiniques, et en particulier les fibres kératiniques humaines telles que les cheveux, avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, azoïque cationique, xanthénique, acridinique azinique, triarylméthane, benzénique nitré ou des colorants naturels.

L'utilisation des colorants directs est très répandue car ils présentent certains avantages par rapport aux précurseurs de colorants d'oxydation et, notamment, une diminution des risques potentiels d'allergie, l'absence de sensibilisation du cheveu due au processus oxydatif et des temps de pose moins longs.

Les colorations obtenues sont cependant temporaires ou semi-permanentes, car la nature des interactions qui lient les colorants directs à la fibre kératinique, et leur désorption de la surface et / ou du coeur de la fibre sont responsables de leur faible puissance tinctoriale et de leur mauvaise tenue aux lavages, aux intempéries, ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

L'utilisateur peut choisir le mode de coloration lui permettant d'obtenir des nuances adaptées à ses besoins en terme de reflets et de ténacité. Cependant, pour effacer les couleurs ainsi obtenues, il doit utiliser des compositions de décoloration comprenant un ou plusieurs agents oxydants ou des réducteurs de type réductone, thiol ou sulfite. Parmi les agents oxydants classiquement utilisés, on peut citer le peroxyde d'hydrogène ou des composés susceptibles de produire du peroxyde d'hydrogène par hydrolyse, comme par exemple le peroxyde d'urée ou les persels tels que les perborates, les persulfates, les percarbonates. L'utilisation de ces agents oxydants présente l'inconvénient d'entraîner une dégradation non négligeable des fibres kératiniques et d'altérer leurs propriétés cosmétiques. Les cheveux ont tendance à devenir rèches, plus difficilement démêlables et plus fragiles. Les réducteurs de type réductone, thiol ou sulfite présentent quant à eux l'inconvénient de ne pas convenir à tous les types de colorants. Ainsi, s'ils permettent d'effacer les colorants azoïques, ils sont inopérants avec les colorants dérivés des anthraquinones.

Le but de la présente invention est de fournir de nouvelles compositions pour la coloration des fibres kératiniques qui ne présentent pas les inconvénients de celles de l'art antérieur. En particulier, le but de la présente invention est de fournir de nouvelles compositions pour la coloration des fibres kératiniques permettant d'obtenir rapidement des colorations avec des reflets intenses et tenaces qui peuvent s'effacer grâce à un agent extérieur qui n'altère pas les fibres kératiniques, comme un agent de pH ou la chaleur par exemple.

Ce but est atteint avec la présente invention qui a pour objet l'utilisation pour la coloration des fibres kératiniques, et en particulier des fibres kératiniques humaines telles que les cheveux, d'une composition comprenant, dans un milieu approprié pour la teinture, au moins un composé choisi parmi les composés de formule (I), les colorants correspondant aux composés de formule (I) dont le cycle A est ouvert et leurs sels d'addition : 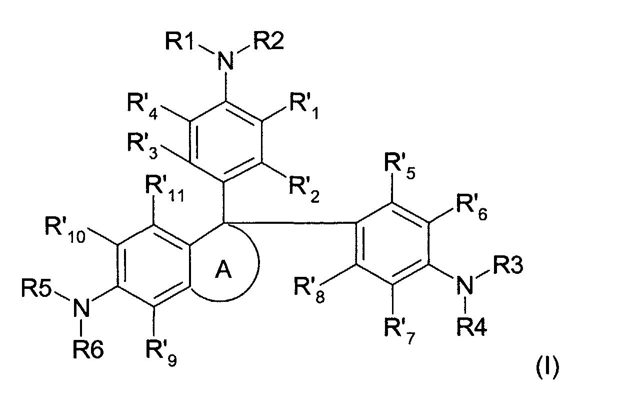 dans laquelle :
- R₁, R₂, R₃, R₄, R₅, R₆ représentent, indépendamment l'un de l'autre :
   - un atome d'hydrogène ;
   - un radical halogéno ;
   - un radical nitro ;
   - un radical alkyle en C₁-C₆ ;
   - un radical hydroxyalkyle en C₁-C₆ ;
   - un radical aminoalkyle en C₁-C₆ ;
   - un radical halogénoalkyle en C₁-C₆ ;
   - un radical alcoxy(C₁-C₃)alkyle en C₁-C₆ ;
   - un radical carboxyalkyle en C₁-C₆ ;
   - un radical sulfoalkyle en C₁-C₆ ;
   - un radical cycloalkyle en C₃-C₁₂ ;
   - un radical aryle en C₆-C₁₈ ;
   - un radical alkyl(C₁-C₆)aryle en C₆-C₁₈ ;
   - un radical hydroxyaryle en C₆-C₁₈ ;
   - un radical aminoaryle en C₆-C₁₈;
   - un radical alcoxy(C₁-C₃)aryle en C₆-C₁₈ ;
ou
- R₁ et R₂ ou R₃ et R₄ ou R₅ et R₆ forment ensemble, avec l'atome d'azote auquel ils sont rattachés, un cycle saturé à 5 ou 6 chaînons, carboné ou contenant un ou plusieurs hétéroatomes ;
- R'₁, R'₂, R'₃, R'₄, R'₅, R'₆, R'₇, R'₈, R'₉, R'₁₀ et R'₁₁ représentent, indépendamment l'un de l'autre :
   - un atome d'hydrogène ;
   - un radical halogéno ;
   - un radical alkyle en C₁-C₆ ;
   - un radical amino ;
   - un radical hydroxyle ;
   - un radical alcoxy en C₁-C₆ ;
   - un radical hydroxyalkyle en C₁-C₆ ;
   - un radical mono ou dialkylamino en C₁-C₆ ;
- A représente un cycle pouvant subir une ouverture en formant un groupement acide.

Par groupement acide, on entend un groupement acide carboxylique, acide sulfonique, acide phosphonique ou acide phosphorique. De préférence, le groupement acide est un groupement acide carboxylique.

La présente invention a également pour objet un procédé de traitement des fibres kératiniques, et en particulier des fibres kératiniques humaines telles que les cheveux, mettant en oeuvre la composition utile dans le cadre de l'invention.

Un autre objet de l'invention est une composition pour la coloration des fibres kératiniques, et en particulier des fibres kératiniques humaines telles que les cheveux, comprenant au moins un composé comportant un cycle pouvant subir une ouverture en formant un groupement acide et / ou le colorant correspondant à ce composé dont le cycle est ouvert et au moins un agent tensioactif.

La présente invention permet en particulier d'obtenir rapidement une coloration des fibres kératiniques avec des reflets intenses et tenaces pouvant être effacée puis reformée tout aussi rapidement. Le procédé d'effaçage et de reformation de la couleur peut être reproduit au moins une fois sans perte substantielle de couleur à l'aide d'un agent de pH ou en agissant sur la température.

Les composés avec un cycle A fermé sont incolores ou faiblement colorés et les composés correspondant à l'ouverture du cycle A pour former un groupement acide sont des espèces colorées et colorantes. En milieu aqueux, il s'établit un équilibre entre les espèces colorées et les espèces non colorées qui dépend du pH et de la température.

Le cycle A est de préférence un cycle à 5 ou 6 chaînons comprenant au moins un hétéroatome choisi de préférence parmi un atome d'oxygène, un atome d'azote, un atome de phosphore.

Le cycle A peut par exemple être un cycle lactone dans le cas des spirolactones, un cycle phosphine dans le cas des phosphonites, un cycle tétrahydropyridine-2-one dans le cas des quinazolones ou un cycle oxozinane dans le cas des oxobenzoaxazines.

Lorsque la composition comprend plusieurs composés choisis parmi les composés de formule (I), les colorants correspondant aux composés de formule (I) dont le cycle A est ouvert et leurs sels d'addition, la couleur des fibres kératiniques obtenue peut aussi être modifiée à l'aide d'un agent de pH ou en agissant sur la température.

Dans le cadre de la présente invention, on entend par radical alkyle (alk) un radical linéaire ou ramifié, par exemple un radical méthyle, éthyle, n-propyle, iso-propyle, n-butyle ou ter-butyle. Un radical alcoxy est un radical alk-O-, un radical mono ou dialkylamino est un radical (alk)ₙN- avec n = 1 ou 2, le radical alkyle étant tel que défini précédemment.

Un radical alkyle substitué est un radical alkyle mono ou polysubstitué. En particulier, un radical hydroxyalkyle est un radical alkyle qui peut être substitué par un ou plusieurs groupements hydroxy, un radical aminoalkyle est un radical alkyle qui peut être substitué par un ou plusieurs groupements amino, un radical halogénoalkyle est un radical alkyle qui peut être substitué par un ou plusieurs groupements halogéno, un radical alcoxyalkyle est un radical qui peut être substitué par un ou plusieurs groupements alcoxy, un radical carboxyalkyle est un radical alkyle qui peut être substitué par un ou plusieurs groupements carboxy, un radical sulfoalkyle est un radical alkyle qui peut être substitué par un ou plusieurs groupements sulfo.

Un radical halogéno désigne un atome d'halogène choisi parmi le chlore, le brome, l'iode et le fluor. Un groupement sulfo est un groupement - SO₃H.

On entend par radical cycloalkyle un radical alkyle dans lequel les atomes de carbone forment un cycle, par exemple un radical cyclohexyle.

On entend par radical aryle un radical carboné dérivé des composés benzéniques condensés ou non condensés, par exemple un radical phényle, anthracényle ou naphtalényle.

Un radical aryle substitué est un radical aryle mono ou polysubstitué. En particulier, un radical alkylaryle est un radical aryle qui peut être substitué par un ou plusieurs groupements alkyle, un radical hydroxyaryle est un radical aryle qui peut être substitué par un ou plusieurs groupements hydroxyle, un radical aminoaryle est un radical aryle qui peut être substitué par un ou plusieurs groupements amino, un radical alcoxyaryle est un radical aryle qui peut être substitué par un ou plusieurs groupements alcoxy.

Un cycle saturé à 5 ou 6 chaînons, carboné ou contenant un ou plusieurs hétéroatomes peut être par exemple un cycle pyrrolidine, un cycle pipéridine, un cycle pipérazine, un cycle morpholine.

Selon un mode de réalisation particulier de l'invention, R₁, R₂, R₃, R₄, R₅, R₆ sont choisis parmi un atome d'hydrogène ; un radical alkyle en C₁-C₆ ; un radical cycloalkyle en C₃-C₁₂ ; un radical aryle en C₆-C₁₈ ; un radical alkyl(C₁-C₆)aryle en C₆-C₁₈. A titre d'exemple, R₁, R₂, R₃, R₄, R₅, R₆ peuvent être un atome d'hydrogène ; un radical méthyle ; un radical éthyle ; un radical n-propyle ; un radical isopropyle ; un radical n-butyle ; un radical ter-butyle.

Selon un mode de réalisation particulier de l'invention, R'₁, R'₂, R'₃, R'₄, R'₅, R'₆, R'₇, R'₈, R'₉, R'₁₀, R'₁₁ sont choisis parmi un atome d'hydrogène ; un radical alkyle en C₁-C₆ ; un radical hydroxyle ; un radical amino ; un radical mono ou dialkylamino en C₁-C₆. A titre d'exemple, R'₁, R'₂, R'₃, R'₄, R'₅, R'₆, R'₇, R'₈, R'₉, R'₁₀, R'₁₁ peuvent être un atome d'hydrogène ; un radical méthyle ; un radical éthyle ; un radical n-propyle ; un radical isopropyle ; un radical n-butyle ; un radical ter-butyle ; un radical hydroxyle ; un radical amino.

Selon un mode de réalisation particulier de l'invention, A est un cycle à 5 ou 6 chaînons comprenant au moins un hétéroatome choisi parmi un atome d'oxygène, un atome d'azote, un atome de phosphore. A titre d'exemple, A peut être :
- un cycle lactone de formule (II) : 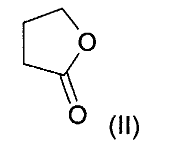
- un cycle phosphine de formule (III) : 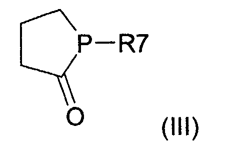
- un cycle tétrahydropyridine-2-one de formule (IV) : 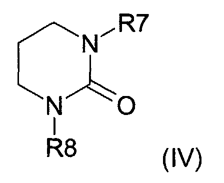
- un cycle oxozinane de formule (V) : 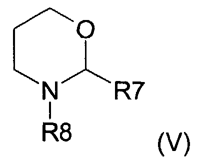
dans lesquelles R₇ et R₈ représentent, indépendamment l'un de l'autre, un atome d'hydrogène ou un radical alkyle en C₁-C₃.

De préférence, A est un cycle lactone de formule (II).

A titre d'exemples de composés de formule (I), on peut citer le composé 3,3-bis-(para-diméthylaminophényl) 6-diméthylamino phtalide ou crystal violet lactone et le composé aminomorpholinorhodamine.

Les composés choisis parmi les composés de formule (I), les colorants correspondant aux composés de formule (I) dont le cycle A est ouvert et leurs sels d'addition représentent en général de 0,0001 à 10 % en poids par rapport au poids total de la composition tinctoriale conforme à l'invention.

D'une manière générale, les sels d'addition des composés de formule (I) et des colorants correspondant aux composés de formule (I) dont le cycle A est ouvert utilisables dans le cadre de l'invention sont notamment choisis parmi les sels d'addition avec un acide tel que les chlorhydrates, les bromhydrates, les sulfates, les méthosulfates, les gluconates, les citrates, les succinates, les tartrates, les lactates, les tosylates, les benzènesulfonates, les phosphates et les acétates et les sels d'addition avec une base telle que la soude, la potasse, l'ammoniaque, les amines ou les alcanolamines.

La composition utile dans le cadre de l'invention peut en outre contenir un ou plusieurs colorants directs pouvant notamment être choisis parmi les colorants nitrés de la série benzénique, les colorants directs azoïques, les colorants directs méthiniques et les colorants naturels. Ces colorants directs peuvent être de nature non ionique, anionique ou cationique.

En présence de colorants directs additionnels dans la composition utile dans le cadre de l'invention, ceux-ci représentent en général de 0,001 à 20 % en poids par rapport au poids total de la composition tinctoriale, de préférence de 0,01 à 10 %.

La composition utile dans le cadre de la présente invention peut en outre comprendre un ou plusieurs colorants d'oxydation choisis parmi les bases d'oxydation et les coupleurs classiquement utilisés en coloration d'oxydation.

En présence de colorants d'oxydation dans la composition utile dans le cadre de l'invention, ceux-ci représentent en général de 0,001 à 20 % en poids par rapport au poids total de la composition tinctoriale, de préférence de 0,01 à 10 %.

La composition utile dans le cadre de l'invention peut en outre comprendre au moins un agent tensioactif.

Le ou les agents tensioactifs utiles dans le cadre de la présente invention peuvent être de type anionique, non-ionique, amphotère ou zwittérionique, cationique.

A titre d'exemple d'agents tensioactifs de type anionique, on peut citer notamment les sels, en particulier les sels alcalins, notamment de sodium, les sels d'ammonium, les sels d'amines, les sels d'aminoalcools ou les sels de magnésium, des composés suivants : les alkylsulfates ; les alkyléthersulfates ; les alkylamidoéthersulfates ; les alkylarylpolyéthersulfates ; les monoglycérides sulfates ; les alkylsulfonates ; les alkylphosphates ; les alkylamidesulfonates ; les alkylarylsulfonates ; les α-oléfine-sulfonates ; les paraffine-sulfonates ; les alkyl(C₆-C₂₄) sulfosuccinates ; les alkyl(C₆-C₂₄) éthersulfosuccinates ; les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates ; les alkylpolyglycoside tartrate et les alkylpolyglycoside sulfosuccinates ; les alkylsulfosuccinamates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

Les agents tensioactifs de type non-ionique sont, eux aussi, des composés bien connus en soi : voir notamment à cet égard "Handbook of Surfactants" par M.R. PORTER, éditions Blackie & Son (Glasgow and London), 1991, pp 116-178. Ainsi, ils peuvent être notamment choisis parmi les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène ; les condensats d'oxyde d'éthylène et de propylène sur des alcools gras ; les amides gras polyéthoxylés ayant de préférence de 2 à 30 moles d'oxyde d'éthylène ; les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose ; les esters d'acides gras du polyéthylèneglycol ; les alkylpolyglycosides ; les dérivés de N-alkyl glucamine ; les oxydes d'amines tels que les oxydes d'alkyl (C₁₀ - C₁₄) amines ou les oxydes de N-acylaminopropylmorpholine.

Les agents tensioactifs de type amphotère ou zwitterionique peuvent être notamment des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant, par exemple carboxylate, sulfonate, sulfate, phosphate ou phosphonate ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO-)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, Cg, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid. A titre d'exemple on peut citer le cocoamphodiacetate commercialisé sous la dénomination commerciale MIRANOL® C2M concentré par la société RHODIA CHIMIE.

Parmi les agents tensioactifs de type cationique, on peut citer en particulier les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium ; les dérivés d'imidazoline ; ou les oxydes d'amines à caractère cationique.

Le ou les agents tensioactifs utiles dans le cadre de l'invention sont de préférence choisis parmi les agents tensioactifs non amides. Par agents tensioactifs non amides, on entend, au sens de la présente invention, des agents tensioactifs qui ne comportent pas de fonction amide.

En présence d'agents tensioactifs dans la composition utile dans le cadre de la présente invention, ceux-ci représentent en général de 0,01 à 40 % en poids par rapport au poids total de la composition tinctoriale, de préférence de 0,5 à 30 %.

Le milieu approprié pour la teinture, appelé aussi support de teinture, est généralement constitué par de l'eau ou par au moins un solvant organique ou par un mélange d'eau et d'au moins un solvant organique. A titre de solvant organique, on peut par exemple citer les cétones tels que l'acétone ; les alcanols en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, et leurs mélanges.

Les composés choisis parmi les composés de formule (I), les colorants correspondant aux composés de formule (I) dont le cycle A est ouvert et leurs sels d'addition sont soit solubles, soit en dispersion dans le support de teinture.

Les solvants sont généralement présents dans des proportions comprises entre 1 et 40 % en poids environ par rapport au poids total de la composition tinctoriale, et encore plus préférentiellement entre 5 et 30 % en poids environ.

La composition utile dans le cadre de l'invention peut également renfermer divers adjuvants utilisés classiquement dans les compositions pour la teinture des cheveux, tels que des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des polymères cationiques ou amphotères, des cations, des silicones volatiles ou non volatiles, modifiées ou non modifiées, des chitosanes ou des dérivés de chitosane, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture utile dans le cadre de l'invention ne soient pas, ou substantiellement pas, altérées par la ou les adjonctions envisagées.

Lorsque les solvants sont constitués par de l'eau ou par un mélange d'eau et d'au moins un solvant organique, le pH de la composition tinctoriale utile dans le cadre de l'invention est généralement compris entre 2 et 12. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux comme par exemple l'acide chlorhydrique, l'acide nitrique, l'acide sulfurique ou les acides organiques comme par exemple les composés comprenant au moins une fonction acide carboxylique comme l'acide acétique, l'acide tartrique, l'acide citrique, l'acide lactique, une fonction acide sulfonique, une fonction acide phosphonique ou une fonction acide phosphorique.

Parmi les agents alcalinisants on peut citer, à titre d'exemple :
- les acides aminés basiques ;
- les carbonates ou les bicarbonates de métaux alcalins ou alcalino-terreux ;
- les silicates ou les métasilicates ;
- les composés de formule (VI) suivante :

   X(OH)ₙ (VI)

   dans laquelle :
   - X représente un ion potassium, lithium, sodium, ammonium N⁺R₉R₁₀R₁₁R₁₂ avec R₉, R₁₀, R₁₁ et R₁₂, identiques ou différents, désignant un radical alkyle en C₂-C₄ lorsque n est égal à 1 ;
   - X représente un atome de magnésium ou de calcium lorsque n est égal à 2 ;
- les composés de formule (VII) suivante : 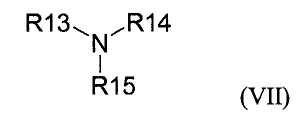 dans laquelle :
   - R₁₃ représente un atome d'hydrogène ; un radical alkyle en C₁-C₆ ; un radical monohydroxyalkyle en C₁-C₆ ; un radical polyhydroxyalkyle en C₂-C₆ ;
   - R₁₄ et R₁₅, identiques ou différents, représentent un atome d'hydrogène ; un radical alkyle en C₁-C₆ ; un radical monohydroxyalkyle en C₁-C₆ ; un radical polyhydroxyalkyle en C₂-C₆;
- les composés de formule (VIII) suivante : 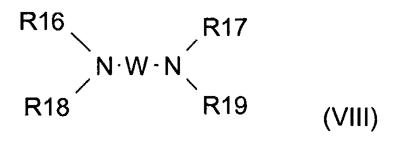 dans laquelle :
   - W est un reste propylène éventuellement substitué par un groupement hydroxyle ou un radical alkyle en C₁-C₄ ;
   - R₁₆, R₁₇, R₁₈ et R₁₉, identiques ou différents, représentent un atome d'hydrogène ; un radical alkyle en C₁-C₄ ou hydroxyalkyle en C₁-C₄.

Au sens de la présente invention, on entend par "acide aminé basique", soit (i) un acide aminé présentant en plus de la fonction amine positionnée en α du groupement carboxyle, un groupement cationique (ou basique) supplémentaire; soit (ii) un acide aminé présentant une chaîne latérale (hydrophile) cationique (ou basique); soit (iii) un acide aminé portant une chaîne latérale constituée d'une base azotée. Ces définitions sont généralement connues et publiées dans les ouvrages de biochimie générale tels que J.H. WEIL (1983) pages 5 et suivantes, Lubert STRYER (1995) page 22, A. LEHNINGER (1993) pages 115-116, DE BOECK-WESMAEL (1994) pages 57-59.

Les acides aminés basiques conformes à l'invention sont choisis de préférence parmi ceux répondant à la formule (D) suivante : 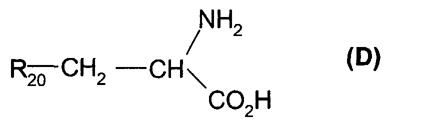 où R₂₀ désigne un groupe choisi parmi :

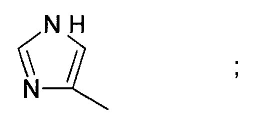

-(CH₂)₃NH₂ ;

-(CH₂)₂NH₂ ;

-(CH₂)₂NHCONH₂ ;

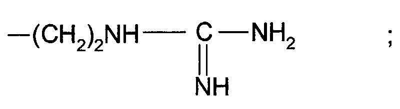

Parmi les composés de formule (D), on peut citer à titre d'exemple l'histidine, la lysine, l'ornithine, la citrulline, l'arginine.

La composition utile dans le cadre de l'invention peut se présenter sous des formes diverses, telles que sous forme de liquides, de crèmes, de gels, ou sous toute autre forme appropriée pour réaliser une coloration des fibres kératiniques, et notamment des cheveux humains.

Le procédé de traitement des fibres kératiniques conforme à l'invention permet d'obtenir une coloration avec des reflets intenses et tenaces qui peut varier en fonction du pH ou de la température et qui peut être effacée et reformée au moins une fois sans perte substantielle de couleur. De préférence, la coloration est modifiée, effacée ou reformée, en ajustant le pH à l'aide d'un agent acidifiant ou d'un agent alcalinisant.

Un procédé préféré de traitement des fibres kératiniques de la présente invention consiste à mettre en oeuvre les étapes suivantes :
a) une composition tinctoriale telle que définie précédemment est appliquée sur les fibres kératiniques pendant un temps de pose suffisant, cette application étant éventuellement suivie d'un rinçage, et le pH est ajusté à l'aide d'un agent acidifiant ou d'un agent alcalinisant en fonction de la coloration souhaitée ;
b) éventuellement la coloration des fibres kératiniques est effacée ou modifiée à l'aide d'un agent acidifiant ou d'un agent alcalinisant appliqué sur les fibres kératiniques ;
ou b') éventuellement la coloration des fibres kératiniques est effacée, modifiée ou reformée au moins une fois à l'aide d'un agent acidifiant ou d'un agent alcalinisant appliqué sur les fibres kératiniques.

Selon un mode de réalisation particulier de l'invention, dans la première étape, l'agent acidifiant ou l'agent alcalinisant est mélangé à la composition tinctoriale avant application sur les fibres kératiniques.

Selon un autre mode de réalisation particulier de l'invention, dans la première étape, l'agent acidifiant ou l'agent alcalinisant est appliqué avant ou après la composition tinctoriale.

Dans le sens de la présente invention, la coloration est effacée lorsque les fibres kératiniques ont retrouvé leur couleur d'origine. La coloration est modifiée lorsque la coloration obtenue est différente de celle obtenue au cours de l'étape précédente. La coloration est reformée lorsque la coloration des fibres kératiniques obtenue est la même que celle qui avait été obtenue au cours d'une étape précédente et qui avait ensuite été modifiée.

Le temps de pose de la composition tinctoriale est généralement compris entre 5 minutes et 1 heure, de préférence entre 15 minutes et 1 heure.

La température d'application est généralement fixée entre la température ambiante et 220°C, de préférence entre la température ambiante et 45 °C.

Les fibres kératiniques peuvent être ou non rincées après application de l'agent acidifiant ou l'agent alcalinisant.

Les agents acidifiants ou alcalinisants sont choisis parmi ceux qui sont décrits ci-dessus.

La présente invention a également pour objet un dispositif à plusieurs compartiments ou kit permettant de mettre en oeuvre le procédé de coloration des fibres kératiniques décrit ci-dessus.

Le dispositif à plusieurs compartiments de l'invention contient dans un premier compartiment une composition tinctoriale telle que définie précédemment et dans un deuxième compartiment un agent acidifiant ou un agent alcalinisant. Dans une autre version, le dispositif à plusieurs compartiments de l'invention contient dans le deuxième compartiment un agent acidifiant et dans un troisième compartiment un agent alcalinisant.

La composition utile dans le cadre de la présente invention comprenant de plus au moins un agent tensioactif telle que définie précédemment est nouvelle et constitue un autre objet de l'invention.

L'exemple qui suit sert à illustrer l'invention sans toutefois présenter un caractère limitatif.

### EXEMPLE

La composition tinctoriale ci-dessous est préparée :

| | composition 1 |
|---|---|
| Crystal Violet Lactone | 1 g |
| Acétone | q.s.p. 100 g |

Cette composition est appliquée sur des mèches de cheveux gris à 90 % de cheveux blancs naturels et permanentés, à raison de 5 g pour 1 g de cheveux, à température ambiante pendant 30 minutes. La couleur est ensuite révélée par application d'une solution d'acide sulfurique 0,1 M sur les mèches. A l'issue de la révélation de la couleur, la mèche est séchée.

Les mèches ainsi colorées peuvent être décolorées très rapidement par application d'une solution de soude 0,1 M. Les mèches retrouvent leurs reflets d'origine.

## Revendications

1. Utilisation pour la coloration des fibres kératiniques, et en particulier des fibres kératiniques humaines telles que les cheveux, d'une composition comprenant, dans un milieu approprié pour la teinture, au moins un composé choisi parmi les composés de formule (I), les colorants correspondant aux composés de formule (I) dont le cycle A est ouvert et leurs sels d'addition : 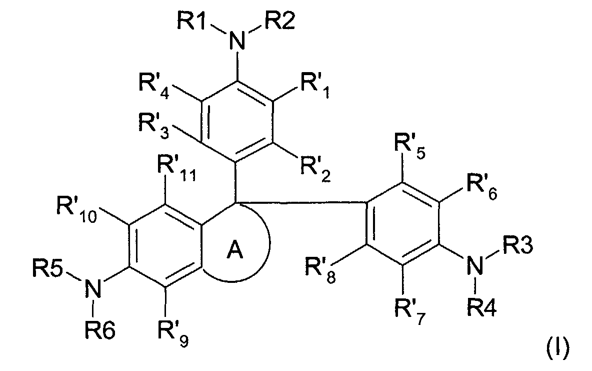 dans laquelle :
• R₁, R₂, R₃, R₄, R₅, R₆ représentent, indépendamment l'un de l'autre :
- un atome d'hydrogène ;
- un radical halogéno ;
- un radical nitro ;
- un radical alkyle en C₁-C₆ ;
- un radical hydroxyalkyle en C₁-C₆ ;
- un radical aminoalkyle en C₁-C₆ ;
- un radical halogénoalkyle en C₁-C₆ ;
- un radical alcoxy(C₁-C₃)alkyle en C₁-C₆ ;
- un radical carboxyalkyle en C₁-C₆ ;
- un radical sulfoalkyle en C₁-C₆ ;
- un radical cycloalkyle en C₃-C₁₂ ;
- un radical aryle en C₆-C₁₈ ;
- un radical alkyl(C₁-C₆)aryle en C₆-C₁₈ ;
- un radical hydroxyaryle en C₆-C₁₈ ;
- un radical aminoaryle en C₆-C₁₈ ;
- un radical alcoxy(C₁-C₃)aryle en C₆-C₁₈ ;
ou
• R₁ et R₂ ou R₃ et R₄ ou R₅ et R₆ forment ensemble, avec l'atome d'azote auquel ils sont rattachés, un cycle saturé à 5 ou 6 chaînons, carboné ou contenant un ou plusieurs hétéroatomes ;
• R'₁, R'₂, R'₃, R'₄, R'₅, R'₆, R'₇, R'₈, R'₉, R'₁₀ et R'₁₁ représentent, indépendamment l'un de l'autre :
- un atome d'hydrogène ;
- un radical halogéno ;
- un radical alkyle en C₁-C₆ ;
- un radical amino ;
- un radical hydroxyle ;
- un radical alcoxy en C₁-C₆ ;
- un radical hydroxyalkyle en C₁-C₆ ;
- un radical mono ou dialkylamino en C₁-C₆ ;
• A représente un cycle pouvant subir une ouverture en formant un groupement acide.

2. Utilisation selon la revendication 1, dans laquelle le composé de formule (I) est tel que R₁, R₂, R₃, R₄, R₅, R₆ sont choisis parmi un atome d'hydrogène ; un radical alkyle en C₁-C₆ ; un radical cycloalkyle en C₃-C₁₂ ; un radical aryle en C₆-C₁₈ ; un radical alkyl(C₁-C₆)aryle en C₆-C₁₈.

3. Utilisation selon la revendication 2, dans laquelle le composé de formule (I) est tel que R₁, R₂, R₃, R₄, R₅, R₆ sont choisis parmi un atome d'hydrogène ; un radical méthyle ; un radical éthyle ; un radical n-propyle ; un radical isopropyle ; un radical n-butyle ; un radical ter-butyle.

4. Utilisation selon l'une quelconque des revendications précédentes, dans laquelle le composé de formule (I) est tel que R'₁, R'₂, R'₃, R'₄, R'₅, R'₆, R'₇, R'₈, R'₉, R'₁₀, R'₁₁ sont choisis parmi un atome d'hydrogène ; un radical alkyle en C₁-C₆ ; un radical hydroxyle ; un radical amino ; un radical mono ou dialkylamino en C₁-C₆.

5. Utilisation selon la revendication 4, dans laquelle le composé de formule (I) est tel que R'₁, R'₂, R'₃, R'₄, R'₅, R'₆, R'₇, R'₈, R'₉, R'₁₀, R'₁₁ sont choisis parmi un atome d'hydrogène ; un radical méthyle ; un radical éthyle ; un radical n-propyle ; un radical isopropyle ; un radical n-butyle ; un radical ter-butyle ; un radical hydroxyle ; un radical amino.

6. Utilisation selon l'une quelconque des revendications précédentes, dans laquelle le composé de formule (I) est tel que A est un cycle à 5 ou 6 chaînons comprenant au moins un hétéroatome.

7. Utilisation selon la revendication 6, dans laquelle le composé de formule (I) est tel que A est choisi parmi :
- un cycle lactone de formule (II) : 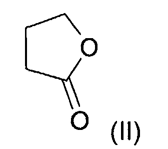
- un cycle phosphine de formule (III) : 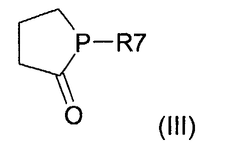
- un cycle tétrahydropyridine-2-one de formule (IV) : 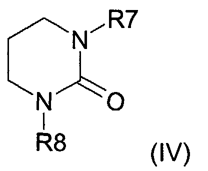
- un cycle oxozinane de formule (V) : 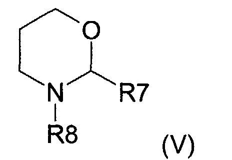
dans lesquelles R₇ et R₈ représentent, indépendamment l'un de l'autre, un atome d'hydrogène ou un radical alkyle en C₁-C₃.

8. Utilisation selon la revendication 7, dans laquelle le composé de formule (I) est tel que A est un cycle lactone de formule (II).

9. Utilisation selon l'une quelconque des revendications précédentes, dans laquelle le ou les composés choisis parmi les composés de formule (I), les colorants correspondant aux composés de formule (I) dont le cycle A est ouvert et leurs sels d'addition représentent de 0,0001 à 10 % en poids par rapport au poids total de la composition.

10. Utilisation selon l'une quelconque des revendications précédentes, comprenant de plus au moins un agent tensioactif.

11. Utilisation selon la revendication 10, dans laquelle le ou les agents tensioactifs sont choisis parmi les agents tensioactifs non amides.

12. Utilisation selon la revendication 10 ou 11, dans laquelle le ou les agents tensioactifs représentent de 0,01 à 40 % en poids par rapport au poids total de la composition.

13. Utilisation selon l'une quelconque des revendications précédentes, dans laquelle le milieu approprié pour la teinture est constitué par de l'eau ou par au moins un solvant organique ou par un mélange d'eau et d'au moins un solvant organique.

14. Utilisation selon la revendication 13, dans laquelle le milieu approprié pour la teinture est constitué par de l'eau ou par un mélange d'eau et d'au moins un solvant organique et le pH est compris entre 2 et 12.

15. Procédé de traitement des fibres kératiniques, et en particulier des fibres kératiniques humaines telles que les cheveux, **caractérisé en ce que** les étapes suivantes sont mises en oeuvre :
a) une composition tinctoriale telle que définie à l'une quelconque des revendications 1 à 14 est appliquée sur les fibres kératiniques pendant un temps de pose suffisant, cette application étant éventuellement suivie d'un rinçage, et le pH est ajusté à l'aide d'un agent acidifiant ou d'un agent alcalinisant en fonction de la coloration souhaitée ;
b) éventuellement la coloration des fibres kératiniques est effacée ou modifiée à l'aide d'un agent acidifiant ou d'un agent alcalinisant appliqué sur les fibres kératiniques ;
ou b') éventuellement la coloration des fibres kératiniques est effacée, modifiée
ou reformée au moins une fois à l'aide d'un agent acidifiant ou d'un agent alcalinisant appliqué sur les fibres kératiniques.

16. Procédé selon la revendication 15, dans lequel, dans la première étape, l'agent acidifiant ou l'agent alcalinisant est mélangé à la composition tinctoriale avant application sur les fibres kératiniques.

17. Procédé selon la revendication 15, dans lequel, dans la première étape, l'agent acidifiant ou l'agent alcalinisant est appliqué avant ou après la composition tinctoriale.

18. Procédé selon l'une quelconque des revendications 15 à 17, dans lequel les fibres kératiniques sont rincées après application de l'agent acidifiant ou l'agent alcalinisant.

19. Procédé selon l'une quelconque des revendications 15 à 18, dans lequel l'agent acidifiant est choisi parmi les acides minéraux tels que l'acide chlorhydrique, l'acide nitrique, l'acide sulfurique ou les acides organiques tels que les composés comprenant au moins une fonction acide carboxylique, une fonction acide sulfonique, une fonction acide phosphonique ou une fonction acide phosphorique.

20. Procédé selon l'une quelconque des revendications 15 à 19, dans lequel l'agent alcalinisant est choisi parmi :
- les acides aminés basiques ;
- les carbonates ou les bicarbonates de métaux alcalins ou alcalino-terreux ;
- les silicates ou les métasilicates ;
- les composés de formule (VI) suivante :
X(OH)ₙ (VI)
dans laquelle :
• X représente un ion potassium, lithium, sodium, ammonium N⁺R₉R₁₀R₁₁R₁₂ avec R₉, R₁₀, R₁₁ et R₁₂, identiques ou différents, désignant un radical alkyle en C₂-C₄ lorsque n est égal à 1 ;
• X représente un atome de magnésium ou de calcium lorsque n est égal à 2 ;
- les composés de formule (VII) suivante : 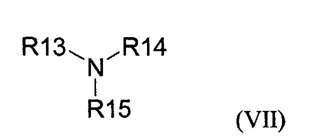 dans laquelle :
• R₁₃ représente un atome d'hydrogène ; un radical alkyle en C₁-C₆ ; un radical monohydroxyalkyle en C₁-C₆ ; un radical polyhydroxyalkyle en C₂-C₆ ;
• R₁₄ et R₁₅, identiques ou différents, représentent un atome d'hydrogène ; un radical alkyle en C₁-C₆ ; un radical monohydroxyalkyle en C₁-C₆ ; un radical polyhydroxyalkyle en C₂-C₆;
- les composés de formule (VIII) suivante : 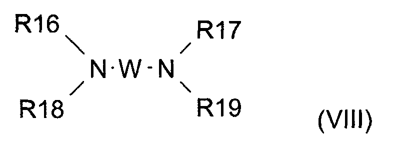 dans laquelle :
• W est un reste propylène éventuellement substitué par un groupement hydroxyle ou un radical alkyle en C₁-C₄ ;
• R₁₆, R₁₇, R₁₈ et R₁₉, identiques ou différents, représentent un atome d'hydrogène ; un radical alkyle en C₁-C₄ ou hydroxyalkyle en C₁-C₄.

21. Dispositif à plusieurs compartiments contenant dans un premier compartiment une composition telle que définie à l'une quelconque des revendications 1 à 14 et dans un deuxième compartiment un agent acidifiant ou un agent alcalinisant.

22. Dispositif selon la revendication 21, contenant dans le deuxième compartiment un agent acidifiant et dans un troisième compartiment un agent alcalinisant.

23. Composition pour la coloration des fibres kératiniques, et en particulier des fibres kératiniques humaines telles que les cheveux, comprenant, dans un milieu approprié pour la teinture, au moins un composé choisi parmi les composés de formule (I) tels que définis à l'une quelconque des revendications 1 à 8, les colorants correspondant aux composés de formule (I) dont le cycle A est ouvert et leurs sels d'addition et au moins un agent tensioactif tel que défini à la revendication 10 ou 11.

24. Composition selon la revendication 23, dans laquelle le ou les composés choisis parmi les composés de formule (I), les colorants correspondant aux composés de formule (I) dont le cycle A est ouvert et leurs sels d'addition représentent de 0,0001 à 10 % en poids par rapport au poids total de la composition.

25. Composition selon la revendication 23 ou 24, dans laquelle le ou les agents tensioactifs représentent de 0,01 à 40 % en poids par rapport au poids total de la composition.
